# EUROPEAN PATENT SPECIFICATION

(11) **EP 0 398 893 B1**
(45) Date of publication and mention of the grant of the patent: **27.10.1993**
(21) Application number: 89900339.6
(22) Date of filing: 30.11.1988
(51) Int. Cl.: A61K 7/22

(54) **PREPARATION FOR DENTAL TREATMENT AND METHOD FOR PRODUCTION OF THE PREPARATION**
ZUBEREITUNG FÜR ZAHNBEHANDLUNG UND VERFAHREN ZUR HERSTELLUNG
PREPARATION POUR TRAITEMENT DENTAIRE ET PROCEDE DE PRODUCTION D'UNE TELLE PREPARATION

(30) Priority: 01.12.1987 SE 8704832
(43) Date of publication of application: 28.11.1990
(73) Proprietor: C.HEDWARD .MEDI-TEAM AB, S-421 66 Västra Frölunda (SE)
(72) Inventor: STRID, Lars, S-413 20 Göteborg (SE); HEDWARD, Christer, S-421 66 Västra Frölunda (SE)
(74) Representative: Mossmark, Anders
(86) International application number: SE8800652
(87) International publication number: WO8905135

## Description

The present invention relates to a preparation for dental treatment and to a method for production of the preparation.

As is known, caries represents an attack on the physiological substance, dentine, which constitutes the tooth substance in humans and animals. If a carious site is not treated, there is a high risk of the attack spreading. Mechanical treatment has been used to a large extent in order to remove the attacked tooth substance. However, it has been found that certain chemical compounds are effective in breaking up caries. A compound known in this connection is sodium hypochlorite.

### Technical problem:

However, the said compound irritates the mucous membranes when it comes into contact with these during spraying of a tooth. Attempts to reduce the aggressive effect of hypochlorite on the mucous membranes have, however, resulted in the caries-detaching effect being reduced, so that it has not been possible to achieve a satisfactory result upon dental treatment.

In FR-A-2 196 784 it is suggested to add an amino compound to the sodium hypochlorite. This will, however, result in the caries detaching effect being reduced, so that it has not been possible to achieve a satisfactory result upon dental treatment.

In order to improve the half-life of a preparation containing sodium hypochlorite and amino acid it is suggested in EP-A-0 224 599 to also add glycine. Thereby an overall significant increase in the half-life will be obtained. Both the amino acid and glycine have a neutral net electric charge.

### Solution:

The preparation according to the invention has the form of a caries-detaching liquid consisting of sodium hypochlorite and nitrogen-containing chemical substances, which consist of three nitrogen-containing compounds with different charge states; neutral and negative net charge and positive net charge. They preferably consist at least partially of amino acids. In the method, the nitrogen-containing compounds are fed in a common stream to a stream of the sodium hypochlorite solution, so that a mixing of the solutions takes place as near as possible ahead of the site where the effect is to be obtained during the dental treatment.

### Advantages:

The present invention provides a preparation which, despite having a strong caries-detaching effect upon treatment, has a limited aggressive action on the mucous membranes.

The method, which is connected with the final production of the preparation, contributes to the said effect of maintaining a good detaching effect with respect to caries but a limited aggressiveness to the mucous membranes.

### Preferred embodiments:

Dentine, the tooth substance, contains amino acids bound to each other in long chains. The side chain of an amino acid can either be neutral or have a negative or a positive net charge. A mixture of three N-chlorinated, organic compounds, preferably amino acids, where one is neutral, one has a negative net charge and the last a positive net charge, therefore gives better results as a caries-detaching substance than does the use of one type of N-chlorinated compound.

The basic idea is that three nitrogen-containing compounds with different charge states are produced and used as a preparation for dental treatment and detachment or caries in mixture with sodium hypochlorite. The organic compounds are expediently present in the form of an acid, a neutral and a basic amino acid.

More precisely, the preparation according to the invention consists partly of sodium hypochlorite which, as mentioned, has a strong detaching effect on the carious substance, and three nitrogen-containing compounds which are preferably amino acids and which, when mixed with the sodium hypochlorite, are N-chlorinated and give compounds containing an active chlorine which, while retaining a caries-detaching property, does not exhibit the aggressiveness of sodium hypochlorite towards mucous membranes.

The chlorination of the amino acid or the organic compound containing an -NH- group takes place with sodium hypochlorite at a pH of about 11 and with an excess of the amino compound. The reaction between sodium hypochlorite and amino compound is extremely fast. However, the resulting N-chlorinated organic compound is not stable, but is broken down relatively quickly.

It is therefore proposed according to the invention that the method for final production of the preparation be connected to the dental treatment by means of the mixing of sodium hypochlorite and the excess of organic compound taking place as near as possible to the carious tooth which is to be treated. This means that these two substances are mixed near the tip of the cannula which is used for injecting the preparation, i.e. as close to the outlet of the cannula as possible.

For the dental treatment an injection nozzle is designed in the form of a cannula. The cannula has a discharge opening, which is intended to be directed towards that area of a subject's set of teeth which is to be treated. Two channels lead to the discharge opening. Both the channels are connected to a pressure source for two liquids, one for each channel. These liquids are designated below by A and B.

The liquids are to be led into the respective channels at such a pressure that a discharge rate suitable for dental treatment is achieved. The pressure can be obtained either by means of pumps, which convey the liquids from containers, or by means of the liquids being fed from pressure containers.

Of the two liquids one is a mixture of organic compounds, as has been mentioned above, and this liquid is designated below by A. The second liquid is sodium hypochlorite, as has also emerged from the above, and this liquid is designated by B. When the two liquids are mixed at the discharge opening, where the two channels run together, the sodium hypochlorite provides for an N-chlorination of the organic -NH- compound, which is supplied in excess, and the stream of the caries-detaching preparation according to the invention is obtained. The caries-detaching effect begins when the preparation flows onto the surface of the tooth, and the detached, carious substance is led away by the stream, as are other undesirable substances on the surface of the tooth, such as plaque.

According to the invention the two liquids A and B are composed in the following manner:

### Basic composition

### A.

The sodium salts of an acid, a neutral and a basic amino acid are mixed in such a ratio that, after chlorination and depending on different reaction rates, they are present as an N-chlorinated derivative in the ratio 1:1:1. The total concentration of the amino acids should be about 0.1-0.2 M. The solution should moreover contain so much sodium chloride that the mixture with solution B (see below) is isotonic and has a pH of about 11.

### B.

Sodium hypochlorite, about 0.014 M in water.

If it is assumed that A and B are brought together at the discharge opening in similar volume amounts per unit of time, then the above concentrations are applicable. If the volume amounts are different, the proportions must be changed accordingly.

### Example:

Solution A can be a mixture of the sodium salts of glutaminic acid, leucine and lysine with a total concentration of about 0.1 M and a pH of about 11.

Solution B is supplied as stated.

## Claims

1. Preparation for dental treatment in the form of a caries-detaching liquid consisting of sodium hypochlorite and nitrogen-containing chemical substances, characterized in that the nitrogen-containing chemical substances consist of three nitrogen-containing compounds with different charge states; neutral and negative net charge and positive net charge, and preferably, in a manner known per se, consisting at least partially of amino acids.

2. Preparation according to patent claim 1, characterized in that the amino acids are incorporated as sodium salts of an acid, a neutral and a basic amino acid and in that this solution is given a pH of about 11.

3. Preparation according to patent claim 1, characterized in that it contains sodium chloride in an amount such that the solution is isotonic at a pH of about 11, the total concentration of the amino acids being preferably about 0.1 M and that of the sodium hypochlorite about 0.014 M in water.

4. Preparation according to patent claim 3, characterized in that the sodium salts consist of glutaminic acid, leucine and lysine.

5. Method for producing the preparation according to any one of patent claims 1-4, characterized in that the nitrogen-containing compounds are fed in a preferably common stream to a stream of the sodium hypochlorite solution, so that a mixing of the solutions takes place as near as possible ahead of the site where the effect is to be obtained during the dental treatment.

## Patentansprüche

1. Zubereitung zur Zahnbehandlung in Form einer Karies-abbauenden Flüssigkeit, bestehend aus Natriumhypochlorit- und Stickstoff-enthaltenden chemischen Substanzen,
dadurch gekennzeichnet,
daß die Stickstoff-enthaltenden chemischen Substanzen aus drei Stickstoff-enthaltenden Verbindungen mit unterschiedlichen Ladungszuständen, nämlich mit neutraler und negativer Nettoladung und positiver Nettoladung, bestehen und daß sie bevorzugt in einer an sich bekannten Weise wenigstens teilweise aus Aminosäuren bestehen.

2. Zubereitung nach Anspruch 1,
dadurch gekennzeichnet,
daß die Aminosäuren als Natriumsalze einer Säure, einer neutralen und einer basischen Aminosäure einbezogen werden und daß diese Lösung einen pH von etwa 11 erhält.

3. Zubereitung nach Anspruch 1,
dadurch gekennzeichnet,
daß sie Natriumchlorid in einer Menge enthält, so daß die Lösung bei einem pH von etwa 11 isotonisch ist, wobei die Gesamtkonzentration der Aminosäuren bevorzugt etwa 0,1 M und die von Natriumhypochlorit etwa 0,014 M in Wasser beträgt.

4. Zubereitung nach Anspruch 3,
dadurch gekennzeichnet,
daß die Natriumsalze aus Glutaminsäure, Leucin und Lysin bestehen.

5. Verfahren zur Herstellung der Zubereitung nach einem der Ansprüche 1 - 4,
dadurch gekennzeichnet,
daß die Stickstoff-enthaltenden Verbindungen in einem bevorzugt gemeinsamen Strahl einem Strahl der Natriumhypochlorit-Lösung zugeführt werden, so daß eine Vermischung der Lösungen so nahe wie möglich vorne an der Stelle, an der die Wirkung während der Zahnbehandlung erhalten werden soll, stattfindet.

## Revendications

1. Préparation pour traitement dentaire sous forme d'un liquide de décollement des caries constitué d'hypochlorite de sodium et de substances chimiques contenant de l'azote, caractérisée en ce que les substances chimiques contenant de l'azote sont constituées de trois composés contenant de l'azote ayant des états de charge différents; charge totale neutre et négative et charge totale positive, et sont de préférence, d'une manière connue en soi, constituées au moins partiellement d'acides aminés.

2. Préparation selon la revendication 1, caractérisée en ce que les acides aminés sont incorporés sous forme de sels sodiques d'un acide aminé acide, d'un acide aminé neutre et d'un acide aminé basique et caractérisée en ce qu'on fixe le pH de cette solution à environ 11.

3. Préparation selon la revendication 1, caractérisée en ce qu'elle contient du chlorure de sodium en une quantité telle que la solution est isotonique à un pH d'environ 11, la concentration totale en acides aminés étant de préférence d'environ 0,1 M et la concentration en hypochlorite de sodium étant de préférence d'environ 0,014 M dans l'eau.

4. Préparation selon la revendication 3, caractérisée en ce que les sels sodiques sont constitués d'acide glutaminique, de leucine et de lysine.

5. Procédé de production de la préparation selon l'une quelconque des revendications 1 à 4, caractérisé en ce que les composés contenant de l'azote sont introduits sous forme d'un courant de préférence commun dans un courant de la solution d'hypochlorite de sodium de sorte qu'un mélange des solutions se produit aussi près que possible avant l'endroit ou l'effet doit être obtenu pendant le traitement dentaire.
